# EUROPÄISCHE PATENTSCHRIFT

(11) **EP 2 014 317 B1**
(45) Veröffentlichungstag und Bekanntmachung des Hinweises auf die Patenterteilung: **27.11.2019**
(21) Anmeldenummer: 08009218.2
(22) Anmeldetag: 20.05.2008
(51) Int. Cl.: A61L 27/04, A61L 27/30, A61L 27/58, A61L 31/02, A61L 31/08, A61L 31/14

(54) **Implantat mit einer oberflächennahen magnesiumhaltigen Diffusionsschicht und dazugehöriges Herstellungsverfahren**
Implant with a near-surface diffusion layer containing magnesium and corresponding production method
Implant doté d'une couche de diffusion contenant du magnésium proche de la surface et son procédé de fabrication

(30) Priorität: 15.06.2007 DE 102007023284
(43) Veröffentlichungstag der Anmeldung: 14.01.2009
(73) Patentinhaber: BIOTRONIK AG, 8180 Bülach (CH)
(72) Erfinder: Müller, Heinz, Dr., 91052 Erlangen (DE); Uggowitzer, Peter, Prof. Dr., 8913 Ottenbach (CH); Löffler, Jörg, Prof. Dr., 5425 Schneisingen (CH)
(74) Vertreter: Keck, Hans-Georg

(56) Entgegenhaltungen:
- EP-A2- 1 891 985
- WO-A1-2004/002369
- WO-A1-2008/035948
- WO-A2-2006/020742
- WO-A2-2006/029375
- DE-A1- 10 118 603
- DE-A1- 10 361 941
- DATABASE WPI Week 200829 Thomson Scientific, London, GB; AN 2008-E15310 XP002688202, & JP 2008 081751 A (UNIV KANSAI) 10. April 2008 (2008-04-10)

## Beschreibung

Die Erfindung betrifft ein orthopädisches oder ein osteosynthetisches Implantat mit einer oberflächennahen magnesiumhaltigen Diffusionsschicht und ein dazugehöriges Herstellungsverfahren.

Unter Implantat wird allgemein jede medizinische Vorrichtung aus einem oder mehreren Werkstoffen verstanden, die absichtlich in den Körper eingebracht wird und entweder teilweise oder ganz von einer Epitheloberfläche bedeckt ist. Implantate lassen sich hinsichtlich der Verwendungsdauer in Temporär- und Permanentimplantate untergliedern. Temporärimplantate verbleiben für einen befristeten Zeitraum im Körper. Permanentimplantate sind für den dauerhaften Verbleib im Körper vorgesehen. Bei Implantaten kann ferner zwischen Prothesen und künstliche Organe unterschieden werden. Eine Prothese ist eine medizinische Vorrichtung, die Gliedmaßen, Organe oder Gewebe des Körpers ersetzt, während unter einem künstlichen Organ eine medizinische Vorrichtung verstanden wird, die teilweise oder ganz die Funktion eines Körperorgans ersetzt. Unter die genannten Definitionen fallen beispielsweise Implantate wie orthopädische oder osteosynthetische Implantate, Herzschrittmacher und Defibrillatoren und vaskuläre Implantate.

Ein Implantatwerkstoff ist ein nicht lebendes Material, das für eine Anwendung in der Medizin eingesetzt wird und mit biologischen Systemen in Wechselwirkung tritt. Grundvoraussetzungen für den Einsatz eines Werkstoffes als Implantatwerkstoff, der bei bestimmungsgemäßen Zweck mit der Körperumgebung in Kontakt steht, ist dessen Körperverträglichkeit (Biokompatibilität). Unter Biokompatibilität wird die Fähigkeit eines Werkstoffes verstanden, in einer spezifischen Anwendung eine angemessene Gewebereaktion hervorzurufen. Dies beinhaltet eine Anpassung der chemischen, physikalischen, biologischen und morphologischen Oberflächeneigenschaften eines Implantates an das Empfängergewebe mit dem Ziel einer klinisch erwünschten Wechselwirkung. Die Biokompatibilität des Implantatswerkstoffs ist weiterhin abhängig vom zeitlichen Ablauf der Reaktion des Biosystems in das implantiert wird. So treten relativ kurzfristig Reizungen und Entzündungen auf, die zu Gewebeveränderungen führen können. Mittel- bis langfristig sind Vergiftungen, Allergien oder gar Krebsbildung Folgen mangelnder Biokompatibilität.

Biologische Systeme reagieren auf Fremdkörper in Abhängigkeit der Eigenschaften des Werkstoffs oder Bauteils in verschiedener Weise. Entsprechend der Reaktion des Biosystems können die Implantatswerkstoffe in bioaktive, bioinerte und degradierbare/resorbierbare Werkstoffe unterteilt werden.

Für die Zwecke der vorliegenden Erfindung sind lediglich metallische Implantatwerkstoffe von Interesse, deren Anwendung beispielsweise in der Osteosynthese, Gelenkersatz, und Dentalchirurgie liegt. Biokompatible Metalle und Metalllegierungen für Permanentimplantate umfassen rostfreie Stähle (z. B. 316L), Kobaltbasislegierungen (z. B. CoCrMo-Gußlegierungen, CoCrMo-Schmiedelegierungen, CoCrWNi-Schmiedelegierungen und CoCrNiMo-Schmiedelegierungen), Reintitan und Titanlegierungen (z. B. cp Titan, TiAl6V4 oder TiAl6Nb7) und Goldlegierungen. Im Bereich biokorrodierbarer Implantate wird der Einsatz von Magnesium oder Reineisen sowie biokorrodierbaren Basislegierungen der Elemente Magnesium, Eisen, Zink, Molybdän und Wolfram angestrebt.

Eine biologische Reaktion auf metallische Elemente hängt ab von der Konzentration, Einwirkdauer und Art der Zuführung. Häufig führt die Gegenwart eines Implantatwerkstoffs zu Entzündungsreaktionen, deren Auslöser mechanische Reize, chemische Stoffe aber auch Stoffwechselprodukte sein können. Der Entzündungsvorgang ist in der Regel begleitet von der Einwanderung neutrophiler Granulozyten und Monozyten durch die Gefäßwände, der Einwanderung von Lymphozyten-Effektorzellen unter Bildung spezifischer Antikörper gegen den Entzündungsreiz, der Aktivierung des Komplementsystems unter Freisetzung von Komplementfaktoren, die als Mediatoren wirken, und letztendlich der Aktivierung der Blutgerinnung. Eine immunologische Reaktion ist meist eng mit der Entzündungsreaktion verbunden und kann zur Sensibilisierung und Allergiebildung führen. Bekannte metallische Allergene umfassen beispielsweise Nickel, Chrom und Kobalt, die auch in vielen chirurgischen Implantaten als Legierungskomponenten Verwendung finden.

Neben seiner Biokompatibilität muss der Implantatswerkstoff natürlich auch immer seine funktionellen Aufgaben erfüllen, nämlich beispielsweise die zumindest temporäre Sicherstellung der mechanischen Integrität des Implantats und gegebenenfalls Abschirmung eines Implantatinnenraumes gegenüber dem umgebenden Medium. Aufgrund dessen lassen sich in der Materialwahl häufig nur Kompromisse zwischen all den zu erfüllenden Anforderungen an das Material realisieren.

Bekannt ist ferner, dass sich ein höheres Maß an Biokompatibilität erreichen lässt, wenn metallische Implantatwerkstoffe mit Beschichtungen aus besonders gewebsverträglichen Materialien versehen werden. Diese Materialien sind zumeist organischer oder synthetisch-polymerer Natur und teils natürlichen Ursprungs. Trotz der erreichten Fortschritte besteht weiterhin ein hoher Bedarf an zumindest alternativen Ansätzen. So besteht z. B. häufig das Problem, dass die aufzubringenden Beschichtungen nur unzureichend haften, insbesondere mit Hinsicht auf die während der Implantation herrschenden Bedingungen.

Weiterhin ist bei der Mehrzahl der medizinischen Implantate eine möglichst feste beziehungsweise sichere Verankerung des Implantats am Implantationsort angestrebt. Grundsätzlich besteht speziell bei metallischen Permanentimplantaten das Problem, dass ein Implantat nicht dauerhaft im Sinne eines biologischen Einwachsens in den Zellverbund integriert werden kann. Dies äußert sich meist in der Ausbildung einer bindegewebsartigen Schicht um das Implantat herum. Diese bindegewebsartige Schicht verhindert den direkten Kontakt der Zellen des umgebenden Gewebes mit dem Implantat; das Implantat ist in diesem Fall nur noch durch Formschluss nicht aber durch eine biologische Adhäsion durch die Zellen verankert.

Im Falle von Permanentimplantaten aus Titan und Titanwerkstoffen wird zwar häufig eine direkte Besiedlung der Implantatsoberfläche mit vitalen Zellen beobachtet, in vielen Fällen tritt aber auch hier die beschriebene bindegewebsartige Schicht auf. Darüber hinaus wird auch bei Titanimplantaten häufig mit fortschreitender Implantationsdauer eine zunehmende Tendenz zur Lockerung und zur Ausbildung von bindegewebsartigen Schichten beobachtet. In seltenen Fällen kommt es auch bei Titanimplantaten an der Grenzfläche zwischen Implantat und Gewebe, speziell bei längerer Implantationsdauer, zu bioinkompatiblen bis zytotoxischen Reaktionen.

Im Falle von anderen Implantatwerkstoffen, wie CoCr-Legierungen oder Implantatstählen sind diese Reaktionen noch wesentlich ausgeprägter.

Auch spezielle Strukturierungen der Implantatoberfläche, welche Besiedlung mit Zellen erleichtern und die Ausbildung von Kontaktstellen zwischen Zelle und Implantatoberfläche fördern sollen, lösen das Problem nur unzureichend. Funktionelle Oberflächen, die durch spezielle Beschichtungen die Ausbildung einer bindegewebsartigen Schicht unterdrücken sollen, sind noch überwiegend im Forschungsstadium; über die Langzeittauglichkeit dieser häufig monomolekularen Schichten gibt es noch keine gesicherten Erkenntnisse.

Werden bei dieser Betrachtung auch die bisher experimentell verfügbaren Implantate aus den sogenannten biologisch abbaubaren Metallen berücksichtigt, so werden auch hier, speziell dann, wenn eine Akkumulation der Abbauprodukte solcher Metalle mit Gewebe in Kontakt stehen, biologische Unverträglichkeiten bis hin zu Nekrosen des Gewebes gefunden. Solche Ergebnisse wurden zum Beispiel bei der Evaluation von Zink und Zink-Basislegierungen als Implantatwerkstoffe für Gefäßimplantate gefunden und sind bei der Verwendung von abbaubaren Implantaten aus Eisen, Eisen-Legierungen, Wolfram und anderen, grundsätzlich im Körper abbaubaren Metallen, zu erwarten, weil der Körper die gebildeten Verbindungen als Fremdkörper identifiziert und eine Fremdkörperreaktion eintritt. Eine solche Reaktion führt in der Regel auch zu einer Aktivierung des Immunsystems verbunden mit einer lokalen Inflammation. Diese Beeinträchtigungen führen auch zu einem verlangsamten Einheilvorgang, verbunden mit einer verzögerten Besiedlung mit Zellen, der Ausbildung von bindegewebsartigen Schichten und einem verzögerten Einheilvorgang.

Durch lonenstrahlimplantation mit Magnesiumionen kann die Oberfläche orthopädischer Implantate aus der Titanlegierung TiAl6V4 derart modifiziert werden, dass die Besiedlung durch humane Osteblasten erleichtert ist (Zreiqat et al.; ,The effect of surface chemistry modification of titanium alloy on signalling pathways in human osteoblasts'; Biomaterials; 2005; S. 7579 - 7586). Die Ionenimplantation führt zu einer Anreicherung von Magnesium auf einen Gehalt von etwa 10 at% bis in eine Tiefe von ca. 60 nm des Materials. Die Eintragtiefe von Magnesium hängt von der Energie der auftreffenden Ionen ab, d.h. es kommt je nach Energieprofil zu einer Anreicherung des Magnesiums in bestimmten oberflächennahen Schichten des Materials. Eine Konversion der Implantatsoberfläche, die bei Titan und Titanlegierungen immer Titandioxid enthält oder aus Titandioxid besteht, findet nicht statt.

WO 2006/020742 A2 beschreibt Implantate, insbesondere Stents, auf die L605 und Mg aufgesputtert werden. Dieses aufgesputterte Material wird dann durch Salpetersäure zum Teil entfernt. Anschließend wird bei 600 °C und 10⁻⁵ torr Magnesium thermisch entfernt. WO 2008/035948 A1 beschreibt in Beispiel 5, dass eine Titanscheibe mit einer Magnesiumlegierung oder mit Reinmagnesium besputtert wird.

Es besteht daher zusammenfassend noch immer ein hohes Bedürfnis, eine bessere Integration eines metallischen Implantats in sein biologisches Umfeld zu ermöglichen. Dies umfasst sowohl die Option einer besseren Besiedlung der Implantatoberfläche und die Vermeidung der Ausbildung von bindegewebsartigen Schichten als auch die daraus resultierende bessere Verankerung eines Implantats am Implantationsort. Natürlich ist eine Verbesserung der Biokompatibilität im Allgemeinen angestrebt. Im Fall von biokorrodierbaren metallischen Implantatwerkstoffen, besteht das Bestreben eine schnellere Besiedlung mit Zellen zu erreichen.

Das erfindungsgemäße Implantat mit
(i) einem eine Basis bildenden Implantatwerkstoff aus einem oder mehreren metallischen Elementen;
(ii) einer die Basis bedeckenden Diffusionsschicht aus zumindest einem der metallischen Elemente des Implantatwerkstoffs und zumindest Magnesium, wobei die Konzentration von Magnesium in der Diffusionsschicht von einer Außenseite des Implantats hin zur Basis abnimmt; und
(iii) optional, einer die Diffusionsschicht bedeckenden Metallschicht aus Magnesium oder einer biokorrodierbaren Magnesiumlegierung;
wobei das Implantat ein orthopädisches oder ein osteosynthetisches Implantat ist, dadurch gekennzeichnet, dass die Diffusionsschicht eine Schichtdicke im Bereich von 20 nm bis 20 µm besitzt, überwindet oder mindert ein oder mehrere der geschilderten Nachteile des Standes der Technik. Das Implantat weist demnach eine mehrschichtige Implantatoberfläche auf, die aus einem eine Basis bildenden permanenten oder biokorrodierbaren metallischen Implantatwerkstoff und einer die Basis bedeckenden Diffusionsschicht aus einem oder mehreren der Elemente des metallischen Implantatwerkstoffs und zumindest Magnesium besteht. Die Diffusionsschicht kann wiederum mit einer Metallschicht aus Magnesium oder einer biokorrodierbaren Magnesiumlegierung bedeckt sein.

Der Erfindung liegt die Erkenntnis zugrunde, dass Implantate aus einem permanenten oder biokorrodierbaren metallischen Implantatwerkstoff mit der erfindungsgemäßen Diffusionsschicht besonders vorteilhaft sind: Sie zeigen eine verbesserte Verankerung des Implantats am Implantationsort, ein hohes Maß an Biokompatibilität, eine verbesserte Besiedlung der Implantatoberfläche und vermeiden die Ausbildung von bindegewebsartigen Schichten. Der metallische Implantatwerkstoff dient nur als Basis, auf die die erfindungsgemäße Diffusionsschicht aufgetragen ist. Diese Diffusionsschicht wird mit einem geeigneten Bearbeitungsverfahren durch Abscheiden von Magnesium oder einer biokorrodierbaren Magnesiumlegierung auf der Basis und gleichzeitiges oder nachfolgendes Umsetzen eines oberflächennahen Teils der Basis aus dem metallischen Implantatwerkstoff mit zumindest Teilen dieser Abscheidung erzeugt. Mit anderen Worten, das aufgetragene Magnesium beziehungsweise die biokorrodierbare Magnesiumlegierung bildet mit einem oberflächennahen Teil des metallischen Implantatwerkstoffs zusammen die Diffusionsschicht aus. Die Diffusionsschicht bildet sich im Zuge des Herstellungsverfahrens durch Diffusionsvorgänge an der Phasengrenze zwischen den beiden metallischen Schichten aus. Das sich bildende Legierungssystem der Diffusionsschicht hängt von vielen Faktoren ab, insbesondere der Temperatur und Behandlungsdauer beim Herstellungsverfahren, der Zusammensetzung der Basis und der Zusammensetzung des zur Erstellung der Diffusionsschicht verwendeten, magnesiumhaltigen Materials. Die entstehende Diffusionsschicht muss dabei nicht zwangsläufig alle Elemente der Basis beziehungsweise der gegebenenfalls zur Herstellung verwendeten Magnesiumlegierung enthalten; jedoch ist zumindest ein metallisches Element der Basis und Magnesium zugegen.

Eine solche Diffusionsschicht besitzt ein sehr hohes Haftvermögen auf der Basis, so dass eine Beschädigung der Struktur beispielsweise im Zuge der Implantation auch bei hoher mechanischer Beanspruchung weitgehend vermieden werden kann.

Ein besonderer Vorteil der Diffusionsschicht besteht aber auch darin, dass die Bestandteile der magnesiumhaltigen Legierung bei Kontakt mit physiologischen Medien oder wenn sie in vivo implantiert werden einem speziellen Degradationsprozess unterliegen. Dabei erfolgt ersten Erkenntnissen der Anmelderin nach keine Auflösung des Metalls im eigentlichen Sinne, sondern ein Umwandlungsprozess. Nach erfolgter Umwandlung bildet der Organismus an dem Ort, an dem sich ursprünglich das Magnesium befand, eine biologische Phase, die im Wesentlichen aus Calcium, Sauerstoff, Phosphor und Kohlenstoff besteht. Das Magnesium selbst wird vollständig aufgelöst und durch die im Körper zur Verfügung stehenden spezifischen Transportsysteme entfernt. Ein genauer Mechanismus des Prozesses ist noch nicht geklärt, jedoch wurde der geschilderte aktive Umbauprozess im Körper an verschiedenen Stellen des Körpers von Versuchstieren durch die Anmelderin nachgewiesen.

Implantate, die aus einer biokorrodierbaren Magnesiumlegierung bestehen, bilden in physiologischer Umgebung durch den geschilderten Umbauprozess eine neue Grenzfläche aus. In bisher allen Untersuchungen der Bio- und Hämokompatibilität haben derartige Implantate überdurchschnittlich gute Ergebnisse erzielt. Der Umbauprozess beginnt unmittelbar mit der Implantation, so dass sich die erfindungsgemäße Modifikation der Implantatoberfläche insbesondere für orthopädische oder osteosynthetische Implantate eignet. Weiterführende Untersuchungen an Explantaten haben darüber hinaus gezeigt, dass es sich bei der Wechselwirkung des Implantats mit seiner physiologischen Umgebung während des Umbauprozesses um ein sogenanntes bioaktives Verhalten dieser Werkstoffe handelt, das heißt, dass von dem Implantat selbst ein Reiz zur Neubildung einer biologischen Phase oder eines biologischen Gewebes ausgeht.

Wird ein solches Implantat nun in sein physiologisches Umfeld eingebracht, führt die Umbaureaktion zu der oben beschriebenen Neubildung einer physiologischen Phase; die Reaktion wandelt im Falle permanenter Implantatwerkstoffe aber nur die sehr dünne äußere Diffusionsschicht um und kommt zum Erliegen, sobald sie in einem Bereich angelangt ist, an dem die Konzentration des eigentlichen, unter physiologischen Bedingungen stabilen Implantatwerkstoffes hoch genug ist. Auf diese Weise entsteht in situ und in vivo ein neuer Verbund aus dem eigentlichen Implantatwerkstoff und dem biologischen System, bei dem die biologisch gebildet Phase sozusagen als Gradientenschicht in den Grundwerkstoff übergeht, was eine besonders gute Verankerung des Implantats erwarten lässt.

Als biokorrodierbar im Sinne der Erfindung werden Legierungen und Elemente bezeichnet, bei denen in physiologischer Umgebung ein Abbau/Umbau stattfindet, so dass der aus dem Werkstoff bestehende Teil des Implantates ganz oder zumindest überwiegend nicht mehr vorhanden ist.

Die Konzentration von Magnesium in der Diffusionsschicht nimmt von einer Außenseite des Implantats hin zur Basis ab. Eine Besonderheit bei einer solchen Beschichtung in Kombination mit einem permanenten Implantatwerkstoff ist, dass der Körper selbst steuert bis zu welcher Tiefe er diese Beschichtung des Implantats umwandelt, was wiederum den verschiedenen Implantationsorten und der dort vorliegenden physiologischen Bedingungen, wie insbesondere pH-Wert und mögliche Anreicherung mit bestimmten Ionen, Rechnung trägt.

Eine solche Implantatoberfläche aus Basis und bedeckender Diffusionsschicht, die zur Ausbildung einer biologisch gebildeten Haftvermittlungsschicht befähigt ist, kann prinzipiell für alle derzeit verwendeten permanenten oder biokorrodierbaren metallischen Implantatwerkstoffe umgesetzt werden, da z. B. durch geeignete thermische Behandlungen entsprechende Diffusionsschichten (oder auch Interdiffusionsschichten) mit allen bisher relevanten Basiswerkstoffen in der Medizintechnik hergestellt werden können. Vorzugsweise ist (a) der metallische Implantatwerkstoff ausgewählt aus der Gruppe der Elemente Titan, Nickel, Eisen, Kobalt, Niob, Zink, Wolfram, Molybdän und Tantal oder (b) der metallische Implantatwerkstoff eine Basislegierung, bei der eines der genannten Elemente eine Hauptkomponente darstellt. Hauptkomponente ist die Legierungskomponente, deren Gewichtsanteil an der Legierung am höchsten ist. Ein Anteil der Hauptkomponente beträgt vorzugsweise mehr als 50 Gew.%, insbesondere mehr als 70 Gew.%. Derartige Legierungen werden hier als Basislegierungen der jeweiligen Elemente bezeichnet.

Besonders bevorzugt ist der metallische Implantatwerkstoff Titan oder eine Titanlegierung, zum Beispiel cp Titan, TiAl6V4, TiAl6Nb7 oder Nitinol. Zum Einen besteht bei Titan eine gewisse Löslichkeit für Magnesium, zum Anderen bilden sich im System Titan/Magnesium keine intermetallischen Phasen aus, was eine durch Verspröden mögliche mechanische Belastung des Werkstoffverbundes vermeidet. Ferner ist durch entsprechende Einstellung der Prozessparameter bei der Herstellung der Diffusionsschicht und durch das Fehlen intermetallischer Phasen eine sehr homogene Einstellung des Konzentrationsgradienten in der Diffusionsschicht zu erwarten. Titan und Titanlegierungen sind in der Regel von einer einige nm dicken Schicht aus Titandioxid bedeckt, die jedoch wesentlich die Reaktivität des Materials und Oberflächeneigenschaften des Implantats prägt. Durch das weiter unten noch näher erläuterte Verfahren zur Oberflächenmodifikation, also zur Erstellung der Diffusionsschicht, wird diese Oxidschicht reduziert. Eine solche reduktive Umsetzung ist nur mit Magnesium und Calcium möglich; jedes andere Element würde also aufgrund der die Diffusion hindernden Oxidschicht nicht zur Bildung einer Diffusionsschicht führen.

Bevorzugt kann weiterhin der metallische Implantatwerkstoff Niob, Tantal, eine Nioblegierung oder eine Tantallegierung sein. Auch die genannten Element und ihre Legierungen bilden keine spröden intermetallischen Phasen aus.

Ferner ist bevorzugt, wenn der Implantatwerkstoff aus Reineisen, einer biokorrodierbaren Eisenlegierung, einer biokorrodierbaren Wolframlegierung, einer biokorrodierbaren Zinklegierung oder einer biokorrodierbaren Molybdänlegierung besteht.

Nach einer weiteren, auch im Zusammenhang mit den zuvor genannten bevorzugten Ausführungsformen realisierbaren Variante enthält die Diffusionsschicht Poren. Im Zuge der metallographischen Auswertung von Versuchen an einem Implantat mit Titanbasis und einer Magnesiumbeschichtung wurde festgestellt, dass es in der Diffusionsschicht zur Ausbildung von Mikroporosität kommt. Eine derartige Oberflächentopographie auf Implantaten ist beispielsweise im orthopädischen Bereich für das Einwachsen von Knochenzellen angestrebt. Die Ursachen der Mikroporosität sind noch nicht abschließend geklärt; vermutlich bedingt die unterschiedliche Diffusion von Titan und Magnesium das Phänomen (Kirkendall-Effekt). Der Kirkendall-Effekt besteht darin, dass sich bei genügend hoher Temperatur bei zwei aneinanderliegenden festen Phasen das Volumen der einen Phase verringert, während sich das Volumen der anderen Phase vergrößert. Oft entstehen in der Phase, die ihr Volumen verringert, auch charakteristische Löcher nahe der Phasengrenze, die sogenannten Kirkendall-Löcher.

Die Poren weisen vorzugsweise eine durchschnittliche Porengröße von 10 nm bis 10 µm auf. Besonders bevorzugt ist dabei (a) eine durchschnittliche Porengröße von 10 nm bis 1 µm und (b) eine durchschnittliche Porengröße von 1 µm bis 10 µm. Variante (a) zeichnet sich dadurch aus, dass die Porengröße besonders günstig für die Besiedlung durch humane Osteoblasten ist. Variante (b) eignet sich insbesondere für die Aufnahme von Wirkstoffen und deren Freisetzung im bestimmungsgemäßen Gebrauch (drug eluting).

Die Diffusionsschicht weist eine Schichtdicke im Bereich von 20 nm bis 20 µm auf. Ist der Implantatwerkstoff Titan oder eine Titanlegierung, so liegt die Schichtdicke vorzugsweise im Bereich von 2 bis 20 µm.

Sofern eine Metallschicht vorhanden ist, besitzt diese vorzugsweise eine Schichtdicke im Bereich von 10 nm bis zu 1 mm, insbesondere 10 nm bis zu 300 µm. Oberhalb von 1 mm Schichtdicke ist die Gefahr einer Embolie durch Wasserstoffentwicklung oder einer Schädigung des Gewebes durch Gasakkumulation zu hoch. Schichtdicken unterhalb von 300 µm sind in jeder Applikation hinsichtlich ihrer Wasserstofffreisetzung als unbedenklich einzustufen.

Die Metallschicht besteht vorzugsweise aus einer biokorrodierbaren Magnesiumlegierung. Unter Magnesiumlegierung wird vorliegend ein metallisches Gefüge verstanden, dessen Hauptkomponente Magnesium ist. Hauptkomponente ist die Legierungskomponente, deren Gewichtsanteil an der Legierung am höchsten ist. Ein Anteil der Hauptkomponente beträgt vorzugsweise mehr als 50 Gew.%, insbesondere mehr als 70 Gew.%. Vorzugsweise enthält die biokorrodierbare Magnesiumlegierung Yttrium und weitere Seltenerdmetalle, da sich eine derartige Legierung aufgrund ihrer physiko-chemischen Eigenschaften und hohen Biokompatibilität, insbesondere auch seiner Abbauprodukte, auszeichnet. Besonders bevorzugt ist eine Magnesiumlegierung der Zusammensetzung Seltenerdmetalle 5,2 - 9,9 Gew.%, davon Yttrium 3,7 - 5,5 Gew.%, und Rest < 1 Gew.%, wobei Magnesium den auf 100 Gew.% fehlenden Anteil an der Legierung einnimmt. Diese Magnesiumlegierung bestätigte bereits in klinischen Versuchen ihre besondere Eignung, d. h. zeigt eine hohe Biokompatibilität, günstige Verarbeitungseigenschaften, gute mechanische Kennwerte und ein für die Einsatzzwecke adäquates Korrosionsverhalten. Unter der Sammelbezeichnung "Seltenerdmetalle" werden vorliegend Scandium (21), Yttrium (39), Lanthan (57) und die 14 auf Lanthan (57) folgenden Elemente, nämlich Cer (58), Praseodym (59), Neodym (60), Promethium (61), Samarium (62), Europium (63), Gadolinium (64), Terbium (65), Dysprosium (66), Holmium (67), Erbium (68), Thulium (69), Ytterbium (70) und Lutetium (71) verstanden. Bevorzugt sind weiterhin Magnesiumlegierungen die bis zu 6 Gew.% Zink enthalten. Besonders bevorzugt ist eine Magnesiumlegierung der Zusammensetzung Yttrium 0,5 - 10 Gew.%, Zink 0,5 - 6 Gew.%, Calcium 0,05 - 1 Gew.%, Mangan 0 - 0,5 Gew.%, Silber 0 - 1 Gew.%, Cer 0 - 1 Gew.% sowie Zirkonium 0 - 1 Gew.% oder Silizium 0 - 0,4 Gew.%, wobei sich die Angaben auf Gew.% an der Legierung beziehen und Magnesium sowie herstellungsbedingte Verunreinigungen den auf 100 Gew.% verbleibenden Restanteil an der Legierung einnehmen. Zink scheint bei der Knochenbildung eine besondere Rolle zu spielen, insbesondere wurde ein spezifischer Effekt auf die Proliferation von osteoblastischen Zellen berichtet. Nach Durchführung des erfindungsgemäßen Verfahrens ist Zink ein Bestandteil der Diffusionsschicht und/oder der Metallschicht.

Die Magnesiumlegierung ist so in ihrer Zusammensetzung zu wählen, dass sie biokorrodierbar ist. Als Prüfmedium zur Testung des Korrosionsverhaltens einer in Frage kommenden Legierung dient künstliches Plasma, wie es nach EN ISO 10993-15:2000 für Biokorrosionsuntersuchungen vorgeschrieben ist (Zusammensetzung NaCl 6,8 g/l, CaCl2 0,2 g/l, KCl 0,4 g/l, MgSO₄ 0,1 g/l, NaHCO₃ 2,2 g/l, Na₂HPO₄ 0,126 g/l, NaH₂PO₄ 0,026 g/l). Eine Probe der zu untersuchenden Legierung wird dazu in einem verschlossenen Probenbehälter mit einer definierten Menge des Prüfmediums bei 37 °C gelagert. In zeitlichen Abständen - abgestimmt auf das zu erwartende Korrosionsverhalten - von wenigen Stunden bis zu mehreren Monaten werden die Proben entnommen und in bekannter Weise auf Korrosionsspuren untersucht. Das künstliche Plasma nach EN ISO 10993-15:2000 entspricht einem blutähnlichen Medium und stellt damit eine Möglichkeit dar, eine physiologische Umgebung im Sinne der Erfindung reproduzierbar nachzustellen.

Die Erfindung kann Anwendung bei allen orthopädischen oder osteosynthetischen Implantaten finden, die aus permanenten oder biokorrodierbaren metallischen Implantatwerkstoffen bestehen oder die zumindest Bauteile aus einem permanenten oder biokorrodierbaren metallischen Implantatwerkstoff aufweisen, die nach der Implantation mit dem umgebenden Gewebe in Kontakt stehen. Das Implantat ist ein orthopädisches oder osteosynthetisches Implantat.

Ein weiterer Aspekt der Erfindung besteht in der Bereitstellung eines Verfahrens zur Herstellung der in vorgenannter Weise an der Implantatoberfläche modifizierten Implantate, wobei die Implantate orthopädische oder osteosynthetische Implantate sind. Das erfindungsgemäße Verfahren umfasst die Schritte:
(i) Bereitstellen eines unbehandelten Implantats mit einem eine Basis bildenden Implantatwerkstoff aus einem oder mehreren metallischen Elementen;
(ii) Kontaktieren der Oberfläche der Basis mit metallischem Magnesium oder einer biokorrodierbaren Magnesiumlegierung, wobei das Kontaktieren durch Eintauchen in eine Schmelze aus Magnesium oder einer biokorrodierbaren Magnesiumlegierung erfolgt; und
(iii) im Anschluss an Schritt (ii), thermische Behandlung des Implantats zumindest im Bereich der Kontaktfläche unter Ausbildung einer die Basis bedeckenden Diffusionsschicht aus zumindest einem der metallischen Elemente des Implantatwerkstoffs und zumindest Magnesium, wobei eine Konzentration von Magnesium in der entstehenden Diffusionsschicht von einer Außenseite des Implantates hin zur Basis abnimmt.

Das erfindungsgemäße Verfahren sieht also vor, ein Implantat mit einer Implantatoberfläche aus einem permanenten oder biokorrodierbaren metallischen Implantatwerkstoff zu veredeln, indem eine Diffusionsschicht aus Teilen dieses Implantatwerkstoffs und Magnesium beziehungsweise der biokorrodierbaren Magnesiumlegierung erzeugt wird.

Das Kontaktieren im Schritt (ii) erfolgt durch Eintauchen in eine Schmelze aus Magnesium oder einer biokorrodierbaren Magnesiumlegierung. Eine Temperatur liegt dann im zu behandelnden Bereich vorzugsweise zwischen Schmelztemperatur und Schmelztemperatur + 300°C.

Schritt (iii) wird so durchgeführt, dass eine Konzentration von Magnesium in der entstehenden Diffusionsschicht von einer Außenseite des Implantats hin zur Basis abnimmt. Diese Diffusionsschicht weist dann einen Konzentrationsgradienten für Magnesium auf, das heißt der Atomanteil/Gewichtsanteil von Magnesium in der die Diffusionsschicht bildenden Legierung nimmt hin zur Basis ab. Eine Schichtdicke der gebildeten Diffusionsschicht hängt zum Einen von der Menge des aufgetragenen Magnesiums/der Magnesiumlegierung ab und zum Anderen vom Umfang der Umsetzung des aufgetragenen Materials mit dem metallischen Implantatwerkstoff im oberflächennahen Bereich der Implantatoberfläche.

Schritt (iii) kann vorzugsweise so durchgeführt werden, dass Poren in der Diffusionsschicht entstehen. Ferner kann Schritt (iii) so durchgeführt werden, dass die entstehende Diffusionsschicht von einer Metallschicht bedeckt ist.

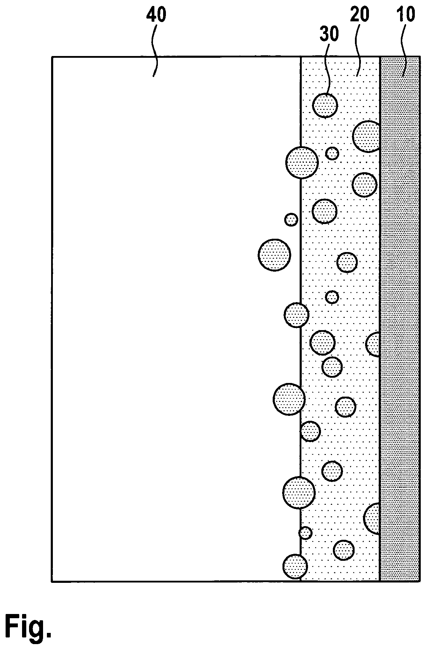

Nachfolgend wird die Erfindung anhand von Ausführungsbeispielen und einer Zeichnung näher erläutert. Die einzige Figur zeigt eine schematische Schnittansicht durch ein Implantat mit einer erfindungsgemäßen Diffusionsschicht.

### Ausführungsbeispiel 1

Ein stabförmiges Implantat aus cp-Titan wurde in einem Vakuumschmelzofen in eine Schmelze aus Reinmagnesium von 750°C getaucht und in dieser für 30 Minuten gehalten. Danach wurde das Implantat aus der Schmelze gezogen und knapp über dem Schmelzbad für 10 Minuten gehalten. Die schmelzmetallurgische Behandlung führte zur Ausbildung einer etwa 7 µm dicken Diffusionsschicht.

Die Beschichtung bestand aus einer ca. 2 µm dicken Magnesiumschicht 10 und einer ca. 5 µm dicken Diffusionsschicht 20 mit abnehmendem Magnesiumgehalt hin zum Inneren des Titanimplantats (Basis 40). In der Diffusionsschicht 20 entstanden Poren 30, mit einer Porengröße von 1 -5 µm (vgl. schematische Schnittdarstellung der Figur erstellt anhand eines Feinschliffes).

Im System Titan/Magnesium erscheint folgender Aufbau der Diffusionsschicht 20 ausgehend von der Basis 40 schlüssig: Es liegt ein einphasiges System aus Titan und Magnesium bis zu einer Grenze von ca. 3 at% Magnesium vor (mit graduellem Anstieg des Mg-Gehalts). Hieran schließt sich ein Bereich mit einem zweiphasigen System an, in dem Titan und Magnesium nebeneinander vorliegen, wobei der Magnesiumanteil kontinuierlich ansteigt.

### Ausführungsbeispiel 2

Eine Magnesiumlegierung der Zusammensetzung (in Gew.%) 2.0%Zn, 0.8%Y und 0.25%Ca wurde bei 750°C in einem Tiegel unter Schutzatmosphäre geschmolzen. Ein stabförmiges Implantat aus cp-Tintan wurde in das Schmelzbad getaucht und in diesem für 60 Minuten gehalten. Danach wurde das Implantat aus der Schmelze gezogen und an Normalatmosphäre abgekühlt. Diese schmelzmetallurgische Behandlung führte zur Ausbildung einer etwa 9 µm dicken Diffusionsschicht mit abnehmendem Magnesiumgehalt hin zum Inneren des Titanimplantats. In der Legierungsschicht entstanden Poren mit einer Porengröße von 1 -5 µm.

## Patentansprüche

1. Implantat mit
(i) einem eine Basis (40) bildenden Implantatwerkstoff aus einem oder mehreren metallischen Elementen;
(ii) einer die Basis (40) bedeckenden Diffusionsschicht (20) aus zumindest einem der metallischen Elemente des Implantatwerkstoffs und zumindest Magnesium, wobei die Konzentration von Magnesium in der Diffusionsschicht (20) von einer Außenseite des Implantats hin zur Basis (40) abnimmt; und
(iii) optional, einer die Diffusionsschicht (20) bedeckenden Metallschicht (10) aus Magnesium oder einer biokorrodierbaren Magnesiumlegierung;
wobei das Implantat ein orthopädisches oder ein osteosynthetisches Implantat ist, **dadurch gekennzeichnet, dass** die Diffusionsschicht (20) eine Schichtdicke im Bereich von 20 nm bis 20 µm besitzt.

2. Implantat nach Anspruch 1, bei dem eine die Diffusionsschicht (20) bedeckende Metallschicht (10) aus Magnesium oder einer biokorrodierbaren Magnesiumlegierung vorliegt.

3. Implantat nach Anspruch 1 oder 2, bei dem (a) der Implantatwerkstoff aus einem oder mehreren Elemente ausgewählt aus der Gruppe Titan, Nickel, Eisen, Kobalt, Niob, Zink, Wolfram, Molybdän und Tantal besteht oder (b) bei dem der Implantatwerkstoff eine Basislegierung ist, bei der eines der genannten Elemente eine Hauptkomponente darstellt.

4. Implantat nach Anspruch 3, bei dem der Implantatwerkstoff aus Titan, einer Titanlegierung, Nitinol, Niob, Tantal, einer Nioblegierung, einer Tantallegierung, Reineisen, einer biokorrodierbaren Eisenlegierung, einer biokorrodierbaren Wolframlegierung, einer biokorrodierbaren Zinklegierung oder einer biokorrodierbaren Molybdänlegierung besteht.

5. Implantat nach einem der vorhergehenden Ansprüche, wobei es sich beim Implantatwerkstoff um einen permanenten Implantatwerkstoff handelt.

6. Implantat nach einem der vorhergehenden Ansprüche, wobei der Implantatwerkstoff Titan, eine Titanlegierung oder Nitinol ist.

7. Implantat nach einem der vorhergehenden Ansprüche, bei dem die Diffusionsschicht (20) Poren (30) enthält und die Poren (30) eine durchschnittliche Porengröße von 10 nm bis 10 µm aufweisen.

8. Implantat nach Anspruch 7, wobei die Poren (30) eine durchschnittliche Porengröße von 1 µm bis 10 µm aufweisen.

9. Implantat nach einem der vorhergehenden Ansprüche, bei dem die Metallschicht (10) eine Schichtdicke im Bereich von 10 nm bis zu 1 mm besitzt.

10. Implantat nach einem der vorhergehenden Ansprüche, bei dem die Diffusionsschicht (20) und/oder die Metallschicht (10) Zink enthalten.

11. Verfahren zur Oberflächenmodifikation eines Implantats, wobei das Implantat ein orthopädisches oder ein osteosynthetisches Implantat ist, umfassend die Schritte:
(i) Bereitstellen eines unbehandelten Implantats mit einem eine Basis (40) bildenden Implantatwerkstoff aus einem oder mehreren metallischen Elementen;
(ii) Kontaktieren der Oberfläche der Basis (40) mit metallischem Magnesium oder einer biokorrodierbaren Magnesiumlegierung, wobei das Kontaktieren durch Eintauchen in eine Schmelze aus Magnesium oder einer biokorrodierbaren Magnesiumlegierung erfolgt; und
(iii) im Anschluss an Schritt (ii), thermische Behandlung des Implantats zumindest im Bereich der Kontaktfläche unter Ausbildung einer die Basis (40) bedeckenden Diffusionsschicht (20) aus zumindest einem der metallischen Elemente des Implantatwerkstoffs und zumindest Magnesium, wobei eine Konzentration von Magnesium in der entstehenden Diffusionsschicht (20) von einer Außenseite des Implantats hin zur Basis (40) abnimmt.

12. Verfahren nach Anspruch 11, bei dem der Schritt (iii) so durchgeführt wird, dass Poren (30) in der Diffusionsschicht (20) entstehen.

13. Verfahren nach einem der Ansprüche 11 bis 12, bei dem der Schritt (iii) so durchgeführt wird, dass die entstehende Diffusionsschicht (20) von einer Metallschicht (10) bedeckt ist.

14. Verfahren nach einem der Ansprüche 11 bis 13, bei dem die biokorrodierbare Magnesiumlegierung aus Schritt (ii) bis zu 6 Gew.% Zink enthält.

## Claims

1. An implant, comprising:
(i) an implant material made of one or more metallic elements forming a base (40);
(ii) a diffusion layer (20) covering the base (40) and made of at least one of the metallic elements of the implant material and at least magnesium, wherein the concentration of magnesium in the diffusion layer (20) decreases from an outer side of the implant toward the base (40); and
(iii) optionally, a metal layer (10) covering the diffusion layer (20) made of magnesium or a biocorrodible magnesium alloy;
wherein the implant is an orthopaedic or an osteosynthetic implant, **characterised in that** the diffusion layer (20) has a layer thickness in the range of from 20 nm to 20 µm.

2. The implant according to claim 1, wherein a metal layer (10) covering the diffusion layer (20) and made of magnesium or a biocorrodible magnesium alloy is provided.

3. The implant according to claim 1 or 2, wherein (a) the implant material consists of one or more elements selected from the group consisting of titanium, nickel, iron, cobalt, niobium, zinc, tungsten, molybdenum, and tantalum, or (b) wherein the implant material is a base alloy, wherein one of said elements is a main component.

4. The implant according to claim 3, wherein the implant material consists of titanium, a titanium alloy, nitinol, niobium, tantalum, a niobium alloy, a tantalum alloy, pure iron, a biocorrodible iron alloy, a biocorrodible tungsten alloy, a biocorrodible zinc alloy, or a biocorrodible molybdenum alloy.

5. The implant according to any one of the preceding claims, wherein the implant material is a permanent implant material.

6. The implant according to any one of the preceding claims, wherein the implant material is titanium, a titanium alloy or nitinol.

7. The implant according to any one of the preceding claims, wherein the diffusion layer (20) contains pores (30) and the pores (30) have an average pore size of 10 nm to 10 µm.

8. The implant according to claim 7, wherein the pores (30) have an average pore size of from 1 µm to 10 µm.

9. The implant according to any one of the preceding claims, wherein the metal layer (10) has a layer thickness in the range of from 10 nm to 1 mm.

10. The implant according to any one of the preceding claims, wherein the diffusion layer (20) and/or the metal layer (10) contain/contains zinc.

11. A method for surface modification of an implant, wherein the implant is an orthopaedic or an osteosynthetic implant, the method comprising the steps:
(i) providing an untreated implant having an implant material, forming a base (40), made of one or more metallic elements;
(ii) contacting the surface of the base (40) using metallic magnesium or a biocorrodible magnesium alloy, wherein the contacting is performed by immersion in a melt of magnesium or a biocorrodible magnesium alloy; and
(iii) following step (ii), thermally treating the implant at least in the area of the contact surface with formation of a diffusion layer (20) covering the base (40) made of at least one of the metallic elements of the implant material and at least magnesium, wherein a concentration of magnesium in the resultant diffusion layer (20) decreases from an outer side of the implant toward the base (40).

12. The method according to claim 11, wherein step (iii) is performed such that pores (30) result in the diffusion layer (20).

13. The method according to any one of claims 11 to 12, wherein step (iii) is performed such that the resultant diffusion layer (20) is covered by a metal layer (10).

14. The method according to any one of claims 11 to 13, wherein the biocorrodible magnesium alloy from step (ii) contains up to 6 % by weight zinc.

## Revendications

1. Implant doté
(i) d'un matériau d'implant constitué d'un ou de plusieurs éléments métalliques formant une base (40) ;
(ii) d'une couche de diffusion (20), recouvrant la base (40), constituée d'au moins un des éléments métalliques du matériau d'implant et d'au moins de magnésium, la concentration en magnésium dans la couche de diffusion (20) diminuant à partir d'un côté extérieur de l'implant jusque vers la base (40) ; et
(iii) éventuellement, d'une couche métallique (10), recouvrant la couche de diffusion (20), à base de magnésium ou d'un alliage de magnésium biocorrodable ;
l'implant étant un implant orthopédique ou ostéosynthétique, **caractérisé en ce que** la couche de diffusion (20) possède une épaisseur de couche dans la plage de 20 nm à 20 µm.

2. Implant selon la revendication 1, chez lequel est présente une couche métallique (10), recouvrant la couche de diffusion (20), à base de magnésium ou d'un alliage de magnésium biocorrodable.

3. Implant selon la revendication 1 ou la revendication 2, chez lequel (a) le matériau d'implant est constitué d'un ou de plusieurs éléments choisis dans le groupe à base de titane, nickel, fer, cobalt, niobium, zinc, tungstène, molybdène et de tantale, ou (b) chez lequel le matériau d'implant est un alliage de base chez lequel un desdits éléments représente une composante principale.

4. Implant selon la revendication 3, chez lequel le matériau d'implant est constitué de titane, d'un alliage de titane, de nitinol, de niobium, de tantale, d'un alliage de niobium, d'un alliage de tantale, de fer pur, d'un alliage de fer biocorrodable, d'un alliage de tungstène biocorrodable, d'un alliage de zinc biocorrodable ou d'un alliage de molybdène biocorrodable.

5. Implant selon l'une des revendications précédentes, dans lequel, dans le cas du matériau d'implant, il s'agit d'un matériau d'implant permanent.

6. Implant selon l'une des revendications précédentes, dans lequel le matériau d'implant est du titane, un alliage de titane ou du nitinol.

7. Implant selon l'une des revendications précédentes, chez lequel la couche de diffusion (20) contient des pores (30) et les pores (30) ont une taille de pore moyenne de 10 nm à 10 µm.

8. Implant selon la revendication 7, dans lequel les pores (30) présentent une taille de pore moyenne de 1 µm à 10 µm.

9. Implant selon l'une des revendications précédentes, chez lequel la couche métallique (10) possède une épaisseur de couche dans la plage de 10 nm à 1 mm.

10. Implant selon l'une des revendications précédentes, chez lequel la couche de diffusion (20) et/ou la couche métallique (10) contiennent du zinc.

11. Procédé de modification de surface d'un implant, l'implant étant un implant orthopédique ou ostéosynthétique, comprenant les étapes :
(i) de fourniture d'un implant non traité doté d'un matériau d'implant formant une base (40) constitué d'un ou de plusieurs éléments métalliques ;
(ii) de mise en contact de la surface de la base (40) avec du magnésium métallique ou un alliage de magnésium biocorrodable, la mise en contact ayant lieu par une immersion dans un produit de fusion de magnésium ou d'un alliage de magnésium biocorrodable ; et
(iii) à la suite de l'étape (ii), de traitement thermique de l'implant au moins dans la zone de la surface de contact, moyennant la formation d'une couche de diffusion (20) recouvrant la base (40) à base d'au moins un des éléments métalliques du matériau d'implant et d'au moins de magnésium, où une concentration en magnésium dans la couche de diffusion (20) en formation diminue à partir d'un côté extérieur de l'implant jusque vers la base (40).

12. Procédé selon la revendication 11, chez lequel l'étape (iii) est effectuée de telle manière que des pores (30) se créent dans la couche de diffusion (20).

13. Procédé selon l'une des revendications 11 à 12, chez lequel l'étape (iii) est effectuée de telle manière que la couche de diffusion (20) en formation est recouverte d'une couche métallique (10).

14. Procédé selon l'une des revendications 11 à 13, chez lequel l'alliage de magnésium biocorrodable de l'étape (ii) contient jusqu'à 6 % en poids de zinc.
